**Europäisches Patentamt**

**European Patent Office**

**Office européen des brevets**

(19)

(11) Veröffentlichungsnummer: **0 257 273 B1**

# EUROPÄISCHE PATENTSCHRIFT

(12)

(45) Veröffentlichungstag der Patentschrift: **10.04.91**

(51) Int. Cl.⁵: **C07C 409/24**, C07C 407/00

(21) Anmeldenummer: **87110140.8**

(22) Anmeldetag: **14.07.87**

(54) Verfahren zur Phlegmatisierung von wasserunlöslichen Peroxycarbonsäuren.

(30) Priorität: **25.08.86 DE 3628263**

(43) Veröffentlichungstag der Anmeldung:
**02.03.88 Patentblatt 88/09**

(45) Bekanntmachung des Hinweises auf die
Patenterteilung:
**10.04.91 Patentblatt 91/15**

(84) Benannte Vertragsstaaten:
**AT BE CH DE ES FR GB IT LI LU NL SE**

(56) Entgegenhaltungen:
**EP-A- 0 127 783**
**GB-A- 2 032 421**
**US-A- 4 287 135**

(73) Patentinhaber: **Degussa Aktiengesellschaft**
**Weissfrauenstrasse 9**
**W-6000 Frankfurt am Main 1(DE)**

(72) Erfinder: **Dankowski, Manfred, Dr.**
**Finkenweg 24**
**W-Mömbris-Königshofen(DE)**
Erfinder: **Hofen, Willi**
**Südring 54**
**W-6458 Rodenbach(DE)**

**Beschreibung**

Die Erfindung betrifft ein Verfahren zur Phlegmatisierung von wasserunlöslichen Peroxycarbonsäuren mit im wesentlichen Natriumsulfat als Phlegmatisierungsmittel, wobei in der Mutterlauge gelöstes Natriumsulfat in gereinigter Form in dem Prozeß zurückgeführt wird.

Peroxycarbonsäuren werden nicht nur als Oxidationsmittel in der organischen Synthese eingesetzt, sondern auch als bleichaktive Komponenten in Wasch- und Reinigungsmitteln, insbesondere solchen für Textilien, da ihre Wirkung schon unterhalb 80 °C auftritt, siehe z.B. EP-A1-0 037 146, US-PS 4,119,660.

Es sind bereits verschiedene Verfahren zur Herstellung von bei Raumtemperatur festen, in Wasser unlöslichen oder wenig löslichen aliphatischen oder aromatischen Mono- und Diperoxycarbonsäuren bekannt. Peroxycarbonsäuren lassen sich z.B. aus den entsprechenden Carbonsäuren mit wäßrigem Wasserstoffperoxid in Gegenwart von Schwefelsäure und einem organischen Lösungsmittel diskontinuierlich, vgl. US-PS 4,172,086, oder kontinuierlich, vgl. EP-B1-0 045 290, herstellen. In Abwesenheit eines organischen Lösungsmittels lassen sich Peroxycarbonsäuren herstellen, wenn man gemäß beispielsweise der US-PS 4,233,235 aliphatische Dicarbonsäuren in konzentrierter Schwefelsäure gelöst oder gemäß beispielsweise US-PS 4,244,884; DE-OS 33 20 497 oder DE-OS 34 18 450 aliphatische oder aromatische Carbonsäuren in Schwefelsäure suspendiert mit Wasserstoffperoxid peroxidiert. Anstelle der Carbonsäuren können teilweise auch deren Anhydride, vgl. DE-OS 34 38 529, mit Wasserstoffperoxid in Gegenwart von Schwefelsäure peroxidiert werden.

Da feste, wasserunlösliche Peroxycarbonsäuren in reinem oder hochkonzentriertem Zustand thermisch und mechanisch sensibel sind, hatte man sich im Zusammenhang mit der Herstellung derselben auch mit der Phlegmatisierung bzw. Stabilisierung befaßt. Eine Phlegmatisierung kann gemäß BE-PS 560 389 insbesondere durch Zusatz von solchen Alkali-, Erdalkali- sowie Erdmetallsalzen starker Mineralsäuren erfolgen, welche Kristallwasser binden, beispielsweise Natriumsulfat, Magnesiumsulfat, Dinatriumorthophosphat, Dinatriumtetraborat, Aluminiumsulfat.

Das Abmischen von hochkonzentrierten Peroxycarbonsäuren mit den entsprechenden Phlegmatisierungsmitteln ist aber aus sicherheitstechnischen Gründen nicht unproblematisch. Demgegenüber vorteilhafter sind Verfahren, bei welchen das Phlegmatisierungsmittel in situ im Anschluß an die Percarbonsäureherstellung in Gegenwart von Schwefelsäure durch Neutralisieren derselben mit Alkali- oder Erdalkalihydroxiden oder Alkalialuminaten oder Alkaliboraten gebildet wird, siehe US-PS 4,287,135; DE-OS 33 20 496; EP-B1-0 045 290. Die Phlegmatisierung von Peroxycarbonsäuren mit in situ-Bildung des sehr gut phlegmatisierenden und in der Anwendung in Bleich- und Reinigungsmitteln nicht störenden Natriumsulfats kann unterhalb der Umwandlungstemperatur von Natriumsulfatdekahydrat in Thenardit, vgl. US-PS 4,287,135, als auch, und dies ist im Hinblick auf die einfachere Trocknung der so phlegmatisierten Peroxycarbonsäuren vorteilhafter, oberhalb desselben erfolgen, vgl. DE-OS 33 20 496; EP-B1-0 045 290.

Bei allen Verfahren zur Phlegmatisierung von wasserunlöslichen Peroxycarbonsäuren mit im wesentlichen Natriumsulfat als Phlegmatisierungsmittel in wäßriger Phase fällt nach der Abtrennung der phlegmatisierten Peroxycarbonsäuren eine Mutterlauge an, welche an Natriumsulfat gesättigt ist und wasserlösliche Verunreinigungen enthält. Bisher wurden die bei Persäureherstellverfahren anfallenden Salzlösungen üblicherweise verworfen. Verfahren zur Phlegmatisierung von Peroxycarbonsäuren können aber nur dann wirtschaftlich durchgeführt und den gestellten Anforderungen an den Umweltschutz gerecht werden, wenn keine natriumsulfathaltige Mutterlauge entsorgt werden muß, sondern das in der Mutterlauge gelöste Natriumsulfat in den Prozeß zurückgeführt werden kann.

Die EP-B1-0 045 290 richtet sich demgemäß auf ein kontinuierliches Verfahren zur Herstellung von Peroxycarbonsäurezusammensetzungen aus den entsprechenden Carbonsäuren und wäßrigem Wasserstoffperoxid in Gegenwart einer konzentrierten starken Säure und eines organischen Lösungsmittels und Natriumsulfat sowie Borsäure als Phlegmatisierungsmittel, wobei die Mutterlauge recycliert wird. Das Phlegmatisierungsmittel wird bei diesem Verfahren in situ aus der anwesenden Schwefelsäure durch Zugabe von Borax und einer Sodaquelle gebildet, wobei diese Umsetzung entweder bei der Phlegmatisierung, das heißt vor der Abtrennung der phlegmatisierten Peroxycarbonsäuren von der Mutterlauge oder nach dieser Abtrennung in der in diesem Fall noch schwefelsauren Mutterlauge erfolgen kann. Bei der ersten Variante wird die an Natriumsulfat und Borsäure gesättigte und nach einer gegebenenfalls erforderlichen Verdampfung eines Teils des Wassers auch Kristalle enthaltende Mutterlauge zur Verdünnung des die Peroxycarbonsäuren enthaltenden schwefelsauren Gemischs vollständig zurückgeführt. Bei der zweiten Variante führt man die durch Neutralisation der Mutterlauge gebildete, kristallisiertes Phlegmatisierungsmittel enthaltende Kristallsuspension zurück.

Bei dem geschilderten bekannten Verfahren werden alle in der wäßrigen Mutterlauge gelösten Verunreinigungen sowie gegebenenfalls als feinteiliger Durchschlag bei der Abtrennung der phlegmatisierten Peroxycarbonsäuren mitgerissene ungelöste Verunreinigungen mit der Mutterlauge recycliert und gegebenenfalls sogar durch Teileindampfung konzentriert. Bei den Verunreinigungen handelt es sich um in den Einsatzstoffen der Percarbonsäureherstellung und/oder der Phlegmatisierung vorhandene Verunreinigungen und/oder in geringer Konzentration vorhandene, im Endprodukt gegebenenfalls störende Verarbeitungshilfsstoffe sowie um Verunreinigungen, welche bei dem Herstellungs- und/oder Phlegmatisierungsverfahren durch chemische Reaktion oder werkstoffbedingt gebildet werden.

Das bekannte Verfahren sieht an keiner Stelle eine Reinigung der Mutterlauge vor. Darüber hinaus wird der Wasserhaushalt des Verfahrens durch sehr teures Eindampfen geregelt. Es baut sich also zunehmend ein höherer Verunreinigungspegel im System auf. Eine durch diese Verunreinigungen katalysierte Zersetzung der Peroxycarbonsäuren in der Phlegmatisierungsstufe läßt sich damit nicht mehr ausschließen. Vom sicherheitstechnischen Standpunkt aus betrachtet ist ein solches Verfahren, zumindest wenn man es im technischen Maßstab durchführen will, selbst dann noch bedenklich oder gar prohibitiv, wenn man aus der Mutterlauge vor der Recyclierung einen Teilstrom ausschleust. Weiterhin nachteilig kommt hinzu, daß nach diesem bekannten Verfahren phlegmatisierte Peroxycarbonsäuren zwangsläufig einen höheren Verunreinigungsgrad aufweisen, was, den praktischen Erfahrungen entsprechend, zu einer geringeren Lagerstabilität führt.

Aufgabe der Erfindung ist demnach, ein Verfahren zur Phlegmatisierung von wasserunlöslichen Peroxycarbonsäuren mit im wesentlichen Natriumsulfat als Phlegmatisierungsmittel in wäßriger Phase und Rückführung des in der Mutterlauge gelösten Natriumsulfats aufzuzeigen, das die Nachteile des bekannten Verfahrens nicht aufweist, sondern durch eine in das Verfahren integrierte Reinigung den Verunreinigungspegel niedrig hält und damit das Verfahren auch im technischen Maßstab sicher und in technisch einfacher Weise durchzuführen gestattet und zu einem lagerstabilen Produkt führt.

Diese Aufgabe wird gelöst durch ein Verfahren zur Phlegmatisierung von wasserunlöslichen Peroxycarbonsäuren mit im wesentlichen Natriumsulfat als Phlegmatisierungsmittel, wobei man die Peroxycarbonsäuren mit dem Phlegmatisierungsmittel in wäßriger Phase miteinander in Kontakt bringt, die phlegmatisierten Peroxycarbonsäuren in bekannter Weise von der Mutterlauge abtrennt und vor dem Trocknen gegebenenfalls konditioniert, und in der Mutterlauge gelöstes Natriumsulfat in den Prozeß zurückführt, das dadurch gekennzeichnet ist, daß man der Mutterlauge nach Abtrennung der phlegmatisierten Peroxycarbonsäuren zur Kristallisation von Natriumsulfatdekahydrat und gegebenenfalls auch Natriumsulfatheptahydrat Wärme entzieht, die auskristallisierten Natriumsulfathydrate von der die Verunreinigungen enthaltenden Ablauge abtrennt und mindestens einen Teil der abgetrennten Natriumsulfathydrate selbst oder nach Überführung derselben in eine wäßrige Lösung und/oder wasserfreies Natriumsulfat in den Prozeß zurückführt.

Die Unteransprüche richten sich auf bevorzugte Ausführungsformen des Verfahrens.

Bei dem erfindungsgemäßen Verfahren fällt nach der Separation der phlegmatisierten Peroxycarbonsäuren durch z.B. Filtrieren oder Zentrifugieren eine im allgemeinen an Natriumsulfat gesättigte Mutterlauge an, welche auch die rohstoff-, verfahrens- und apparatebedingten im wesentlichen in der Mutterlauge gelösten Verunreinigungen enthält. Durch kontinuierliches oder stufenweises Abkühlen der Mutterlauge auf Temperaturen unterhalb 32,4 °C, dem Umwandlungspunkt von Thenardit in Natriumsulfatdekahydrat, kristallisiert Natriumsulfatdekahydrat und bei tieferen Temperaturen, insbesondere unter etwa 12 °C, auch das wenig stabile Natriumsulfatheptahydrat aus, wobei das Heptahydrat in Gegenwart von Feuchtigkeit leicht in das Dekahydrat übergeht, siehe Ullmanns Enzyklopädie der technischen Chemie, 12. Band, Seite 682, Urban u. Schwarzenberg, 1960. Unterhalb des kryohydratischen Punktes der Natriumsulfat enthaltenden wäßrigen Mutterlauge, der je nach Anwesenheit und Konzentration weiterer gelöster Bestandteile wenige Grad unter 0 °C liegt, fällt kein Natriumsulfatdekahydrat/-heptahydrat mehr aus. Nach Abtrennung der auskristallisierten Natriumsulfathydrate durch Filtration oder Zentrifugieren fällt eine an Natriumsulfat stark, je nach Kristallisationsbedingungen bis auf 3 - 5 Gewichtsprozent Natriumsulfat, abgereicherte und an Verunreinigungen stark angereicherte Ablauge an. Diese Ablauge, welche außer geringen Mengen an Natriumsulfat und den Verunreinigungen gegebenenfalls noch etwas Wasserstoffperoxid, Schwefelsäure und Caro'sche Säure sowie gelöste und/oder bei der Separation mitgerissene Spuren an Percarbonsäuren enthält, kann im allgemeinen direkt einer biologischen Klärstufe zugeführt und dort entsorgt werden.

Die bei der Kristallisation gewonnenen Natriumsulfathydrate - hierunter wird das Dekahydrat sowie das wenig stabile Heptahydrat verstanden - sind nach ihrer Abtrennung von der Mutterlauge ausreichend rein. Ohne Risiko einer katalytischen Zersetzung des Aktivsauerstoffs, das heißt des Wasserstoffperoxids, der Caro'schen Säure und/oder Peroxycarbonsäuren, oder Beeinträchtigung der Lagerstabilität der phlegmatisierten Peroxycarbonsäuren können die so gereinigten Natriumsulfate direkt oder nach Überführung derselben in eine wäßrige, insbesondere gesättigte Lösung und/oder wasserfreies Natriumsulfat in den Prozeß zurückgeführt werden. Das in der Mutterlauge gelöste Natriumsulfat wird somit nicht nur in den Prozeß zurückgeführt, sondern es werden auch praktisch alle im Phlegmatisierungsprozeß vorhandenen Verunreinigungen, darunter auch die besonders kritischen Schwermetallspuren, mit der leicht entsorgbaren Ablauge aus dem System ausgetragen.

Das erfindungsgemäße Verfahren mit der es kennzeichnenden Kristallisation und Rückführung des zunächst in der Mutterlauge gelösten Natriumsulfats ist zur Phlegmatisierung von auf bekannten Wegen, insbesondere den zum Stand der Technik angeführten, hergestellten Peroxycarbonsäuren geeignet. Es können nicht nur zunächst isolierte, gegebenenfalls filterfeuchte Peroxycarbonsäuren in wäßriger Phase mit Natriumsulfat, wovon ein Teil aus den aus der Mutterlauge auskristallisierten Natriumsulfathydraten stammt, in Kontakt gebracht und danach als phlegmatisierte Peroxycarbonsäuren von der Mutterlauge abgetrennt werden, sondern bevorzugt auch solche Peroxycarbonsäuren, welche sich noch im wäßrigen Reaktionsgemisch aus ihrer Herstellung befinden.

Ganz besonders vorteilhaft ist es, die Peroxycarbonsäuren nach ihrer Herstellung aus den entsprechenden Carbonsäuren oder Anhydriden und Wasserstoffperoxid in Gegenwart von Schwefelsäure und gegebenenfalls organischen Lösungsmitteln und Verarbeitungshilfsstoffen direkt in der wäßrigen Phase des Reaktionsgemischs, das auch Schwefelsäure enthält, vor oder nach Abtrennung des gegebenenfalls anwesenden organischen Lösungsmittels, mit Natriumsulfat in Kontakt bringt, wobei man mindestens einen Teil des zur Phlegmatisierung erforderlichen Natriumsulfats durch in situ-Bildung aus der anwesenden Schwefelsäure und einer alkalisch wirkenden Natriumverbindung, bevorzugt Natriumhydroxid, erzeugt und mindestens ein Teil des Natriumsulfats erfindungsgemäß rückgeführtes Natriumsulfat ist. Bei dieser Ausführungsform leitet man aus der Mutterlauge isolierte Natriumsulfathydrate, vorzugsweise in Form einer gesättigten wäßrigen Lösung, in das wäßrig-schwefelsaure, die Peroxycarbonsäuren enthaltende Reaktionsgemisch und überführt im Anschluß hieran die Schwefelsäure durch Neutralisation auf einen pH-Wert von 2 bis 6 in Natriumsulfat.

Mit dem erfindungsgemäßen Verfahren kann man in technisch einfacher und sicherer Weise wasserunlösliche Peroxycarbonsäuren vor oder nach ihrer Abtrennung vom Reaktionsgemisch ihrer Herstellung mit im wesentlichen Natriumsulfat phlegmatisieren. Das Merkmal "im wesentlichen Natriumsulfat" besagt, daß in geringerer Menge als Natriumsulfat auch andere für Peroxycarbonsäuren bekannte Phlegmatisierungsmittel anwesend sein können, insbesondere Borsäure, Alkali-, Erdalkali- und Erdmetallsulfate. Der Fachmann wird durch einfache Versuche feststellen, in welcher Menge er diese anderen Phlegmatisierungsmittel, ohne das erfindungsgemäße Verfahren hinsichtlich Sicherheit des Verfahrens und Stabilität des Produktes zu stören, einsetzen kann und ob bzw. in welchem Umfang diese bei der Kristallisation der Natriumsulfathydrate ebenfalls auskristallisieren und/oder er die Ablauge gegebenenfalls vor ihrer Entsorgung nachbehandeln muß.

Die Erfindung ist geeignet zur Phlegmatisierung von wasserunlöslichen aromatischen und aliphatischen Peroxycarbonsäuren, insbesondere Monoperoxy- und Diperoxycarbonsäuren, welche sich von aliphatischen geradkettigen oder verzweigten Mono- oder Dicarbonsäuren mit 6 bis 20, bevorzugt 7 bis 14 Kohlenstoffatomen oder aromatischen Mono- oder Dicarbonsäuren mit 7 bis 12 Kohlenstoffatomen, insbesondere Benzol- und Naphthalindicarbonsäuren, ableiten.
Unter "wasserunlöslich" werden auch solche Peroxycarbonsäuren verstanden, welche eine Wasserlöslichkeit unter etwa 5,0 Gewichtsprozent aufweisen. Besonders bevorzugt werden aliphatische alpha, omega-Diperoxydicarbonsäuren mit 10 bis 14 Kohlenstoffatomen, insbesondere Diperoxydodecandisäure.

Die Peroxycarbonsäuren bringt man in wäßriger Phase mit einer solchen Menge an Natriumsulfat in Kontakt, daß die danach in üblicher Weise von der Mutterlauge abgetrennten und in üblicher Weise, bevorzugt bei 30 °C bis 60 °C, getrockneten phlegmatisierten Peroxycarbonsäuren etwa 3 bis 90 Gewichtsprozent Natriumsulfat enthalten. Bevorzugt führt man die Phlegmatisierung in Gegenwart von in der wäßrigen Phase gelöstem und darin suspendiertem Natriumsulfat durch. Das suspendierte Natriumsulfat kann hierbei als Rohstoff zugegeben und/oder in situ bei der Phlegmatisierung aus anwesender Schwefelsäure gebildet worden sein und/oder es handelt sich um aus der Mutterlauge gewonnenes, rückgeführtes Natriumsulfat.

Die Phlegmatisierung und Abtrennung der phlegmatisierten Peroxycarbonsäuren von der Mutterlauge führt man bevorzugt bei Temperaturen oberhalb 32,4 °C und unterhalb solcher Temperaturen, bei welchen eine merkliche Zersetzung der Peroxycarbonsäuren stattfindet, bevorzugt unterhalb 45 °C, aus. Bei der bevorzugten Ausführungsform bei Temperaturen zwischen 32,4 °C und 45 °C liegt das Phlegmatisierungsmittel als Thenardit vor, und eine Trocknung der gegebenenfalls zuvor noch konditionierten phlegmatisierten Peroxycarbonsäuren bereitet keine Schwierigkeiten. Trennt man dagegen die phlegmatisierten Peroxycarbonsäuren bei Temperaturen unterhalb des Umwandlungspunktes von Thenardit in Natriumsulfatdekahydrat ab, können die genaue Einstellung des Gehalts an Phlegmatisierungsmittel wegen gegebenenfalls auftretender Übersättigungen sowie die Trocknung Probleme bereiten.

Die Kristallisation der Natriumsulfathydrate aus der Mutterlauge führt man vorzugsweise in einer speziell auf diese Kristallisation abgestimmten Art und Weise und dazu geeigneten Apparatur durch. Die Löslichkeit von Natriumsulfat in Wasser fällt im Bereich von etwa 32 °C bis etwa 10 °C derart steil ab, daß infolge starker Übersättigungen zum Beispiel an den Kühlwänden eines Wärmeaustauschers Verkrustungen entstehen, bedingt durch die hohen Wachstumsgeschwindigkeiten und große Keimbildungsraten. Selbst bei Strömungsgeschwindigkeiten im Wärmeaustauscher von 2 m/s und mehr sind solche Verkrustungen an Rohrwänden normaler Rauhigkeit noch möglich.

Es wurde nun gefunden, daß sich diese Schwierigkeiten vermeiden lassen, wenn man, bevorzugt im oberen Temperaturbereich bis etwa +10 °C herunter, die Abführung der Kristallisationswärme und Absenkung der Temperatur der Mutterlauge durch eine Verdampfungskühlung im Vakuum ein- oder mehrstufig, diskontinuierlich oder kontinuierlich, bewerkstelligt. Mittels einer geeigneten Vakkumerzeugung senkt man dabei den Druck im vorzugsweise mit einem Rückflußkondensator ausgestatteten Kristallisationsbehälter schrittweise ab und bringt so die Mutterlauge zum Sieden; dadurch sinkt die Temperatur sehr schnell ab, und im wesentlichen fällt Glaubersalz aus.

Im unteren Temperaturbereich, bevorzugt von etwa +10 °C bis unter 0 °C nahe des kryohydratisohen Punktes, entzieht man der Mutterlauge jedoch die Wärme bevorzugt über Wärmeautauscher, wobei man dies ohne Verkrustungsgefahr mit hinreichend glatten Wärmeaustauscherflächen, beispielsweise polierten Edelstahl- oder Emailrohren, und ausreichend hoher Strömungsgeschwindigkeit durchführen kann. Die Abkühlung und damit die Kristallisationsgeschwindigkeit wird bevorzugt so gesteuert, daß die auskristallisierten Natriumsulfathydrate praktisch keine Mutterlauge und damit keine Verunreinigungen einschließen.

Bei einer weiteren bevorzugten Ausführungsform entzieht man der Mutterlauge zunächst im oberen Temperaturbereich die Wärme durch Verdampfungskühlung im Vakuum und anschließend im unteren Temperaturbereich durch Inbetriebnahme von Wärmeaustauschern.

Die diskontinuierliche Kühlungskristallisation durch Verdampfungskühlung, gegebenenfalls in Kombination mit Wärmeaustauschern, erfolgt ein-, zwei- oder mehrstufig. Bei mehrstufiger Arbeitsweise senkt man die Temperatur der beispielsweise oberhalb 32,4 °C von den phlegmatisierten Peroxycarbonsäuren abgetrennten, an Natriumsulfat gesättigten Mutterlauge in der ersten Stufe auf etwa 25 - 27 °C ab, separiert die kristallhaltige Suspension, bevorzugt auf einer Zentrifuge, und führt die an Natriumsulfat abgereicherte Mutterlauge der zweiten Stufe zu; die zweite Stufe der Abkühlung und Kristallisation beendet man bei etwa 15 °C, dann kühlt man die Mutterlauge nach Seperation in einer dritten Stufe bis auf ca. 0 °C ab, wobei ab etwa 10 °C vorteilhafterweise die Wärme durch Wärmeaustauscher dem System entzogen wird. Die nach der dritten Stufe nach der Separation erhaltene Mutterlauge kann man ohne Probleme, beispielsweise in einer biologischen Kläranlage, entsorgen.

Auf eine dritte Stufe kann verzichtet werden, wenn man bei der zweiten Stufe weiter abgereicherte Mutterlauge oder Ablauge zur ausreichenden Verdünnung der durch Abkühlung sich bildenden Kristallsuspension zusetzt und dann bis auf 0 °C oder darunter abkühlt.

Sorgt man während der Abkühlphase der Mutterlauge für einen kontinuierlichen Kristallaustrag, etwa mittels einer Suspensionsaustragspumpe, so kann die Abkühlung einstufig bis auf unter 0 °C durchgehend vollzogen werden. Ab etwa +10 °C empfiehlt sich, die Wärme über wirksame Wärmeaustauscher abzuführen, da bei tiefen Temperaturen eine Vakuum-Siedekühlung nicht mehr wirtschaftlich arbeitet.

Bei einer weiteren, für eine kontinuierliche Phlegmatisierung im technischen Maßstab besonders bevorzugten Ausführungsform kristallisiert man die Natriumsulfathydrate kontinuierlich bei etwa konstanter Temperatur, bevorzugt unterhalb +10 °C und oberhalb des kryohydratischen Punktes und ganz besonders bei etwa 0 °C, wobei man dem mit wirksamen Kühleinrichtungen ausgestatteten Kristallisationsbehälter, in welchem sich zunächst eine bei der Kristallisationstemperatur an Natriumsulfat gesättigte Mutterlauge befindet, kontinuierlich eine bei höherer Temperatur gesättigte Mutterlauge zuführt und kontinuierlich die entstehende Kristallsuspension abzieht und durch Zentrifugieren oder Filtrieren das kristallisierte Salz von der Ablauge trennt.

Um die rheologischen Eigenschaften der Kristallsuspension im Hinblick auf eine bessere Handhabbarkeit zu verändern und gegebenenfalls die Verbackungsneigung der abgetrennten Natriumsulfathydrate zu erniedrigen, kann man bei ihrer Kristallisation in geringer Menge Kristallisationshilfsmittel einsetzen, soweit diese den eigentlichen Phlegmatisierungsprozeß und die phlegmatisierten Peroxycarbonsäuren nicht stören. Der Fachmann wird solche Hilfsmittel in Vorversuchen auf ihre Eignung testen.

Das aus der Mutterlauge isolierte Glaubersalz (Natriumsulfatdekahydrat, gegebenenfalls mit Anteilen an Natriumsulfatheptahydrat) ist sehr rein und enthält unter 4 ppm Fe und unter 1 ppm Cu.

Die an Natriumsulfat abgereicherte Ablauge enthält, wenn man die erfindungsgemäße Phlegmatisierung in der wäßrigschwefelsauren Phase eines Reaktionsgemisches nach in bekannter Weise erfolgter Peroxycarbonsäureherstellung durchführt und von im Handel erhältlichen Rohstoffen ausgeht, im allgemeinen mehr als 4 ppm Fe und mehr als 1 ppm Cu.

Die in der Ablauge enthaltenen Verunreinigungen und insbesondere die darin gelösten kritischen Schwermetallspuren werden bei dem erfindungsgemäßen Verfahren dem System vollständig entzogen und stellen damit kein Sicherheitsrisiko dar, wohingegen bei dem bisher bekannten Verfahren durch die Rückführung der Mutterlauge der Verunreinigungspegel ständig ansteigt und damit ein sicherer Betrieb nicht mehr gewährleistet werden kann.

Bei dem erfindungsgemäßen Verfahren entfällt gegenüber dem bisher bekannten Verfahren auch eine thermische Belastung der noch Aktivsauerstoff enthaltenden Mutterlauge.

Der Schwermetallgehalt der erfindungsgemäß phlegmatisierten Peroxycarbonsäuren liegt im allgemeinen unter 2 ppm.

Auch nach fünf-maligem Recyclieren des gesamten aus der Mutterlauge isolierten Natriumsulfats weisen die phlegmatisierten Peroxycarbonsäuren Schwermetallgehalte von im allgemeinen deutlich unter 5 ppm auf.

Die Lagerstabilität der erfindungsgemäß phlegmatisierten Peroxycarbonsäuren ändert sich durch die Rückführung des Natriumsulfats praktisch nicht und entspricht derjenigen von solchen Produkten, welche ohne Rückführung der Mutterlauge in wäßriger Phase phlegmatisiert worden sind.

Das von der Ablauge abgetrennte Salz, im wesentlichen Natriumsulfatdekahydrat, kann direkt in die Phlegmatisierung zurückgeführt werden. Vorteilhaft ist jedoch, dieses Salz zuvor durch Aufschmelzen, bevorzugt bei 32,4 °C bis 45 °C, in eine an Natriumsulfat gesättigte wäßrige Lösung und darin suspendiertes wasserfreies Natriumsulfat umzuwandeln und nach Abtrennung der festen von der flüssigen Phase, durch z.B. Zentrifugieren oder Filtrieren, mindestens einen Teil der Lösung und/oder mindestens einen Teil des wasserfreien Natriumsulfats in den Phlegmatisierungsprozeß zurückzuführen. Dieser Verfahrensschritt ist insbesondere dann vorteilhaft, wenn man Peroxycarbonsäuren in einer wäßrig-schwefelsauren Phase, wie sie bei bekannten Peroxycarbonsäure-Herstellungsverfahren nach erfolgter Peroxidation vorliegt, mit in situ-Bildung von Natriumsulfat oberhalb 32,4 °C phlegmatisieren will: Die durch Aufschmelzen der erfindungsgemäß kristallisierten Natriumsulfathydrate erhaltene gesättigte und von ungelöstem $Na_2SO_4$ befreite Lösung gibt man hierbei zur Verdünnung in die wäßrig-schwefelsaure, Peroxycarbonsäure enthaltende Phase, anschließend bildet man in bekannter Weise durch Neutralisation der Schwefelsäure mit einer alkalisch wirkenden Natriumverbindung, bevorzugt Natriumhydroxid, in situ Natriumsulfat und konditioniert bei Bedarf die so phlegmatisierte Peroxycarbonsäure mit aus der Mutterlaugenaufarbeitung erhaltenem wasserfreien Natriumsulfat. Dieses Konditionieren kann vor oder nach der Abtrennung der phlegmatisierten Peroxycarbonsäuren von der Mutterlauge erfolgen, wobei eine Konditionierung vor der Abtrennung bevorzugt ist.

Kennzeichnend an der zuvor dargestellten Verfahrensweise ist, daß man das die wasserlöslichen Peroxycarbonsäuren enthaltende wäßrig-schwefelsaure Reaktionsmedium vor der exotherm verlaufenden in situ-Bildung von Natriumsulfat ausreichend mit zurückgeführter $Na_2SO_4$-Lösung verdünnt. Hierdurch wird nicht nur die Viskosität der Suspension herabgesetzt, sondern auch die Abführung der Neutralisationswärme erleichtert, wodurch das Verfahren einfacher und vor allem sicherer durchführbar wird. Im Anschluß an die Neutralisation und bevorzugt vor der Abtrennung der phlegmatisierten Peroxycarbonsäuren kann man dann das aus der Mutterlaugenaufarbeitung kommende wasserfreie Natriumsulfat ganz oder teilweise zur Konditionierung, d.h. zur genauen Einstellung des gewünschten Phlegmatisierungsgrades, zumischen.

Der technische Fortschritt des erfindungsgemäßen Verfahrens liegt darin, daß es nun erstmals möglich ist, die wäßrige Mutterlauge aus der Phlegmatisierung von Peroxycarbonsäuren so aufzuarbeiten, daß das in ihr gelöste Natriumsulfat in gereinigter Form in die Phlegmatisierung zurückgeführt wird, und das Verfahren technisch einfacher und insbesondere sicherer, auch im technischen Maßstab, betrieben werden kann und nur eine wenig belastete Ablauge entsorgt werden muß.

Die Erfindung wird anhand der Beispiele näher erläutert.

Beispiel 1

Aufarbeitung einer Mutterlauge aus der Herstellung und Phlegmatisierung von Diperoxydodecandisäure (DPDDA) gemäß Beispiel 6 der DE-OS 33 20 497.

In den Behälter eines Kristallisationssystems, ausgestattet mit einem wirksamen Rückflußkondensator, einer Suspensions-Umwälzpumpe, Einrichtungen für die Vakuumerzeugung, einem außenlie-

genden Rohrbündelwärmeaustauscher mit Kühlein- richtung, einer SuspensionsAustragspumpe sowie den notwendigen Zu- und Abführungsrohren und Sicherheitseinrichtungen füllt man 616 kg aufzuarbeitende, bei 40 °C an Natriumsulfat gesättigte Mutterlauge, welche auch geringe Mengen an Wasserstoffperoxid, Schwefelsäure und gegebenenfalls Caro'scher Säure und Verunreinigungen aus dem Prozeß enthält. Mittels einer Vakuumpumpe senkt man den Druck im System schrittweise soweit ab, daß die etwa 40 °C warme Mutterlauge zu sieden beginnt und am Kondensator ein Kondensatrücklauf erzeugt wird; hierbei ist der außenliegende Wärmeautauscher noch nicht im Eingriff. Während des nun einsetzenden Temperaturabfalls entsteht im Kristallisationsbehälter eine Kristallsuspension, die über die Suspensions-Umwälzpumpe in Schwebe gehalten wird. Über eine Suspensions-Austragspumpe leitet man kontinuierlich die Kristallsuspension einer Zentrifuge zu und trennt dort die Kristalle von der nun an Natriumsulfat abgereicherten Mutterlauge, welche man in einem Zwischenbehälter auffängt und von dort zurück in den Kristallisationsbehälter pumpt. Ab etwa 10 °C nimmt man den außenliegenden Wärmeaustauscher, der mit einem Kältemittel gekühlt wird, in Betrieb und senkt die Temperatur durch Umwälzen der Kristallsuspension über den Wärmeaustauscher weiter auf ca. 0 °C ab. Die Strömungsgeschwindigkeit in den glatten Rohren des Wärmeaustauschers beträgt 2 m/sec.

Nach beendeter Kristallisation erhält man auf der Zentrifuge 483kg Natriumsulfatdekahydrat gegebenenfalls mit etwas Heptahydrat. Im Zwischenbehälter bzw. Kristallisationsbehälter erhält man insgesamt 133 kg Ablauge mit ca. 4 Gewichtsprozent Natriumsulfat sowie den angereicherten Verunreinigungen.

Beispiel 2

Überführung des Natriumsulfatdekahydrats in eine gesättigte Lösung und wasserfreies Natriumsulfat:

In einen beheizbaren und mit Mischeinrichtung versehenen Schmelzbehälter füllt man 483 kg des in Beispiel 1 aus einer Mutterlauge zurückgewonnenen Natriumsulfatdekahydrats und schmilzt bei etwa 40 °C auf. Die entstehende Suspension wird über eine Austragspumpe einer Zentrifuge zugeführt. Nach Separation erhält man 413 kg gesättigte reine Natriumsulfatlösung und 70 kg reines wasserfreies Na$_2$SO$_4$ (fest).

Beispiel 3

Phlegmatisierung von Diperoxydodecandisäure:

In eine Oxidationsmischung, bestehend aus 48 kg Wasserstoffperoxid (50 gew.-%ig), 96 kg Schwefelsäure (96 gew.-%ig) und 11 kg recyclisierte Natriumsulfatlösung (6 gew.-%ig) sowie 0,15 kg Tri-n-Octylphosphanoxid, dosiert man 54 kg Dodecandisäure ein und erhitzt 2 - 6 h unter Rühren auf 60 °C

Nach Abkühlen auf 40 °C versetzt man den Ansatz bei dieser Temperatur mit 260 kg einer gemäß Beispiel 2 aus einem vorhergehenden Ansatz erhaltenen Natriumsulfatlösung (30 gew.-%ig) und neutralisiert anschließend mit 250 kg Natriumhydroxidlösung (30 gew.-%ig) bis zum Erreichen des pH- Wertes von 3,5 und konditioniert dann mit 71 kg recycliertem wasserfreien Natriumsulfat, erhalten gemäß Beispiel 2 aus einem vorhergehenden Ansatz. Anschließend separiert man und trocknet. Man erhält die phlegmatisierte 35 gew.-%ige Persäure in einer Ausbeute von 90 %.

**Ansprüche**

1. Verfahren zur Phlegmatisierung von wasserun- löslichen Peroxycarbonsäuren mit im wesentli- chen Natriumsulfat als Phlegmatisierungsmittel, wobei man die Peroxycarbonsäuren mit dem Phlegmatisierungsmittel in wäßriger Phase miteinander in Kontakt bringt, die phlegmatisierten Peroxycarbonsäuren in bekannter Weise von der Mutterlauge abtrennt und vor dem Trocknen gegebenenfalls konditioniert,und in der Mutterlauge gelöstes Natriumsulfat in den Prozeß zurückführt, dadurch gekennzeichnet , daß man der Mutterlauge nach Abtrennung der phlegmatisierten Peroxycarbonsäuren zur Kristallisation von Natriumsulfatdekahydrat und gegebenenfalls auch Natriumsulfatheptahydrat Wärme entzieht, die auskristallisierten Na- triumsulfathydrate von der die Verunreinigun- gen enthaltenden Ablauge abtrennt und mindestens einen Teil der abgetrennten Natriumsulfathydrate selbst oder nach Überführung derselben in eine wäßrige Lösung und/oder wasserfreies Natriumsulfat in den Prozeß zurückführt.

2. Verfahren nach Anspruch 1, dadurch gekennzeichnet , daß man wasserunlösliche Monoperoxy- oder Diperoxycarbonsäuren, welche sich von aliphatischen oder aromatischen Mono- oder Dicarbonsäuren ableiten, phlegmatisiert.

3. Verfahren nach Anspruch 1 oder 2, dadurch gekennzeichnet , daß man die Peroxycarbonsäuren mit einer solchen Menge an Natriumsulfat in Kontakt bringt, daß die abgetrennten und in üblicher Weise getrockneten phlegmatisierten Peroxycarbonsäuren etwa 3 Gewichtsprozent bis 90 Gewichtsprozent Natriumsulfat enthalten.

4. Verfahren nach einem der Ansprüche 1 bis 3, dadurch gekennzeichnet , daß man die Phlegmatisierung und Abtrennung der phlegmatisierten Peroxycarbonsäuren von der Mutterlauge bei Temperaturen oberhalb des Umwandlungspunktes von Natriumsulfatdekahydrat zu Thenardit und unterhalb solcher Temperaturen, bei welchen eine merkliche Zersetzung der Peroxycarbonsäuren stattfindet, bevorzugt oberhalb 32,4 °C und unterhalb 45 °C, durchführt.

5. Verfahren nach einem der Ansprüche 1 bis 4, dadurch gekennzeichnet , daß man die Phlegmatisierung der Peroxycarbonsäuren in Gegenwart von in Wasser gelöstem und suspendiertem Natriumsulfat durchführt.

6. Verfahren nach einem der Ansprüche 1 bis 5, dadurch gekennzeichnet , daß man die Peroxycarbonsäuren in wäßriger Phase, welche Schwefelsäure enthält, mit Natriumsulfat in Kontakt bringt, wobei man mindestens einen Teil des zur Phlegmatisierung erforderlichen Natriumsulfats durch in situ-Bildung aus der anwesenden Schwefelsäure und einer alkalisch wirkenden Natriumverbindung, bevorzugt Natriumhydroxid, erzeugt.

7. Verfahren nach einem der Ansprüche 1 bis 6, dadurch gekennzeichnet ,daß man zur Kristallisation der Natriumsulfathydrate der Mutterlauge durch Verdampfungskühlung im Vakuum Wärme entzieht.

8. Verfahren nach Anspruch 7, dadurch gekennzeichnet , daß man die Verdampfungskühlung im oberen Temperaturbereich, bevorzugt zwischen der Temperatur, bei welcher man die Phlegmatisierung durchführt und etwa +10 °C, einsetzt.

9. Verfahren nach Anspruch 7 oder 8, dadurch gekennzeichnet , daß man die Kristallisation der Natriumsulfathydrate zwei- oder mehrstufig durchführt, wobei man nach jeder Stufe die auskristallisierten Natriumsulfathydrate von der daran abgereicherten Mutterlauge abtrennt und letztere der folgenden Stufe zur weiteren Temperaturabsenkung der Mutterlauge und Kristallisation der Natriumsulfathydrate zuführt.

10. Verfahren nach einem der Ansprüche 1 bis 6, dadurch gekennzeichnet, daß man im unteren Temperaturbereich, bevorzugt ab etwa +10 °C bis unter 0 °C nahe des kryohydratischen Punktes, zur Kristallisation der Natriumsulfathydrate der Mutterlauge Wärme über Wärmeaustauscher entzieht.

11. Verfahren nach einem der Ansprüche 7 bis 9 und 10, dadurch gekennzeichnet , daß man der Mutterlauge im oberen Temperaturbereich Wärme zunächst durch Verdampfungskühlung im Vakuum und anschließend im unteren Temperaturbereich über Wärmeaustauscher entzieht.

12. Verfahren nach einem der Ansprüche 1 bis 8 oder 10 bis 11, dadurch gekennzeichnet , daß man die Kristallisation einstufig durchführt, wobei man dem mit wirksamer Kühleinrichtung ausgestatteten Kristallisationsbehälter kontinierlich Kristallsuspension entnimmt, die Kristalle abtrennt und die so an Natriumsulfat abgereicherte Mutterlauge in den Kristallisationsbehälter zurückführt.

13. Verfahren nach einem der Ansprüche 1 bis 12, dadurch gekennzeichnet , daß man die Kristallisation der Natriumsulfathydrate aus der Mutterlauge bei etwa konstanter Temperatur, bevorzugt unterhalb +10 °C und oberhalb des kryohydratischen Punktes, durchführt, indem man dem mit wirksamer Verdampfungskühleinrichtung und/oder Wärmeaustauscher ausgestatteten Kristallisationsgefäß, enthaltend eine bei der gewählten Kristallisationstemperatur an Natriumsulfat etwa gesättigte Mutterlauge, kontinuierlich eine oberhalb der Kristallisationstemperatur an Natriumsulfat gesättigte Mutterlauge zuführt und kontinuierlich gebildete Natriumsulfatdekahydrat- und/oder Natriumsulfatheptahydrat-Kristallsuspension abzieht und die Kristalle von der Ablauge trennt.

14. Verfahren nach einem der Ansprüche 1 bis 13, dadurch gekennzeichnet , daß man die von der Ablauge abgetrennten Natriumsulfathydrate durch Aufschmelzen am oder oberhalb des Umwandlungspunktes des Natriumsulfatdekahydrat in Thenardit, bevorzugt bei 32,4 °C bis 45 °C, in eine an Natriumsulfat gesättigte Lösung und darin suspendiertes wasserfreies Natriumsulfat überführt, das suspendierte Natriumsulfat in bekannter Weise von der Lösung abtrennt und mindestens einen Teil der Lösung und/oder des wasserfreien Natriumsulfats in den Phlegmatisierungsprozeß zurückführt.

15. Verfahren nach den Ansprüchen 6 und 14, dadurch gekennzeichnet , daß man die aus den Natriumsulfathydraten erhaltene, an Natriumsulfat gesättigte Lösung der die zu phlegmatisierenden Peroxycarbonsäuren enthaltenden wäßrig-schwefelsauren Phase zufügt, die Schwefelsäure in bekannter Weise in Natriumsulfat überführt und das aus den Natriumsulfathydraten erhaltene wasserfreie Natriumsulfat ganz oder teilweise zur Konditionierung der phlegmatisierten Peroxycarbonsäuren vor oder nach der Abtrennung derselben von der Mutterlauge zufügt.

16. Verfahren nach einem der Ansprüche 1 bis 15, dadurch gekennzeichnet , daß neben Natriumsulfat noch andere Phlegmatisierungsmittel, insbesondere Borsäure, Alkali-, Erdalkali- und Erdmetallsulfate anwesend sind.

## Claims

1. A process for phlegmatizing water-insoluble peroxycarboxylic acids, mainly with sodium sulphate as phlegmatizing agent, in which the peroxycarboxylic acids are brought into contact with the phlegmatizing agent in the aqueous phase, the phlegmatized peroxy carboxylic acids are separated from the mother liquor in known manner and optionally conditioned before drying, and sodium sulphate dissolved in the mother liquor is returned to the process, characterised in that after separation of the phlegmatized peroxycarboxylic acids, heat is withdrawn from the mother liquor to crystallize sodium sulphate decahydrate and optionally also sodium sulphate heptahydrate, the crystallized sodium sulphate hydrates are separated from the waste liquor containing the impurities and at least part of the separated sodium sulphate hydrates is returned to the process, either as such or after conversion into an aqueous solution and/or anhydrous sodium sulphate.

2. A process according to Claim 1, characterised in that water-insoluble monoperoxy- or diperoxycarboxylic acids derived from aliphatic or aromatic mono- or dicarboxylic acids are phlegmatized.

3. A process according to Claim 1 or Claim 2, characterised in that the peroxycarboxylic acids are brought into contact with such a quantity of sodium sulphate that the phlegmatized peroxycarboxylic acids which have been separated and dried in the usual manner contain from about 3% by weight to 90% by weight of sodium sulphate.

4. A process according to one of the Claims 1 to 3, characterised in that phlegmatization and separation of the phlegmatized carboxylic acids from the mother liquor are carried out at temperatures above the conversion point of sodium sulphate decahydrate into thenardite and below temperatures at which a marked decomposition of the peroxycarboxylic acids takes place, preferably at temperatures above 32.4° C and below 45° C.

5. A process according to one of the Claims 1 to 4, characterised in that phlegmatization of the peroxycarboxylic acids is carried out in the presence of sodium sulphate dissolved and suspended in water.

6. A process according to one of the Claims 1 to 5, characterised in that the peroxycarboxylic acids are brought into contact with sodium sulphate in an aqueous phase containing sulphuric acid, at least part of the sodium sulphate required for phlegmatization being produced by in situ formation from the sulphuric acid present and a sodium compound which is alkaline in reaction, preferably sodium hydroxide.

7. A process according to one of the Claims 1 to 6, characterised in that heat is withdrawn from the mother liquor by evaporation cooling under vacuum to crystallize the sodium sulphate hydrates.

8. A process according to Claim 7, characterised in that the evaporation cooling is begun in the upper temperature region, preferably between the temperature at which phlegmatization is carried out and about +10° C.

9. A process according to Claim 7 or Claim 8, characterised in that crystallization of the sodium sulphate hydrate is carried out in two or more stages, the crystallized sodium sulphate hydrates being separated after each stage from the mother liquor which has become concentrated therewith, and the mother liquor being transferred to the following stage for further lowering of the temperature of the mother liquor and crystallization of the sodium sulphate hydrates.

10. A process according to one of the Claims 1 to 6, characterized in that heat is withdrawn from the mother liquor by means of heat exchangers in the lower temperature region, preferably from about $+10°C$ to below $0°C$, close to the cryohydratic point, for crystallizing the sodium sulphate hydrates.

11. A process according to one of the Claims 7 to 9 and 10, characterised in that heat is first withdrawn from the mother liquor in the upper temperature region by evaporation cooling under vacuum and then in the lower temperature region by means of heat exchangers.

12. A process according to one of the Claims 1 to 8 or 10 to 11, characterised in that crystallization is carried out in a single stage, crystal suspension being continuously removed from the crystallization container which is equipped with an effective cooling device, the crystals are separated and the mother liquor thus depleted of sodium sulphate is returned to the crystallization container.

13. A process according to one of the Claims 1 to 12, characterised in that crystallization of the sodium sulphate hydrates from the mother liquor is carried out at approximately constant temperature, preferably below $+10°C$ and above the cryohydratic point, by continuously supplying a mother liquor saturated with sodium sulphate above the crystallization temperature to the crystallization vessel which is equipped with an effective evaporation cooling device and/or heat exchanger and contains a mother liquor substantially saturated with sodium sulphate at the chosen crystallization temperature and continuously removing the crystal suspension of sodium sulphate decahydrate and/or sodium sulphate heptahydrate formed and separating the crystals from the waste liquor.

14. A process according to one of the Claims 1 to 13, characterised in that the sodium sulphate hydrates separated from the waste liquor are converted into a solution saturated with sodium sulphate and containing anhydrous sodium sulphate in suspension by melting the sodium sulphate hydrates at or above the conversion point of sodium sulphate decahydrate into thenardite, preferably at $32.4°C$ to $45°C$, the suspended sodium sulphate is separated from the solution in known manner and at least part of the solution and/or of the anhydrous sodium sulphate is returned to the phlegmatization process.

15. A process according to Claims 6 and 14, characterised in that the saturated sodium sulphate solution obtained from the sodium sulphate hydrates is added to the aqueous sulphuric acid phase containing the peroxycarboxylic acids to be phlegmatized, the sulphuric acid is converted into sodium sulphate in known manner and all or part of the anhydrous sodium sulphate obtained from the sodium sulphate hydrates is added for conditioning the phlegmatized peroxycarboxylic acids before or after separation of the latter from the mother liquor.

16. A process according to one of the Claims 1 to 15, characterised in that other phlegmatizing agents are present in addition to sodium sulphate, in particular boric acid and alkali metal, alkaline earth metal and earth metal sulphates.

## Revendications

1. Procédé de stabilisation des acides peroxycarboxyliques insolubles dans l'eau avec principalement du sulfate de sodium comme agent de stabilisation, dans lequel on met en contact les acides peroxycarboxyliques avec l'agent de stabilisation en phase aqueuse, on sépare d'une façon connue en soi les acides peroxycarboxyliques stabilisés de la lessive-mère, et le cas échéant on conditionne avant le séchage, et l'on recycle le sulfate de sodium dissous dans la lessive-mère vers le process, caractérisé en ce qu'on extrait la chaleur de la lessive-mère après séparation des acides peroxycarboxyliques stabilisés pour obtenir la cristallisation du sulfate de sodium décahydrate et le cas échéant également du sulfate de sodium heptahydrate, on sépare les sulfates de sodium d'au moins une partie du sulfate de sodium hydratés ayant recristallisé de la lessive résiduaire contenant les impuretés, et on recycle tel quel ou après transfert de celle-ci dans une solution aqueuse et/ou du sulfate de sodium anhydre vers le process.

2. Procédé selon la revendication 1, caractérisé en ce qu'on stabilise des acides monoperoxy carboxyliques ou des acides diperoxycarboxyliques insolubles dans l'eau, lesquels se séparent des acides mono ou dicarboxyliques aliphtatiques ou aromatiques.

3. Procédé selon l'une quelconque des revendications 1 à 2, caractérisé en ce qu'on met en contact les acides peroxycarboxyliques avec une quantité de sulfate de sodium telle que les acides peroxycarboxyliques séparés et de façon usuelle séchés, stabilisés, contiennent entre 3 % et 90 % en poids de sulfate de sodium.

4. Procédé selon l'une quelconque des revendications 1 à 3, caractérisé en ce qu'on réalise la stabilisation et la séparation des acides peroxycarboxyliques stabilisés en provenance de la lessive-mère à des températures situées au-dessus du point de transformation du sulfate de sodium décahydrate en Thernadite, et au-dessous des températures où s'effectue une décomposition sensible des acides peroxycarboxyliques, de préférence audessus de 32,4°C et au-dessous de 45°C.

5. Procédé conforme à l'une quelconque des revendications 1 à 4, caractérisé en ce qu'on réalise la stabilisation des acides peroxycarboxyliques en présence de sulfate de sodium dissous et en suspension dans l'eau.

6. Procédé selon l'une quelconque des revendications 1 à 6, caractérisé en ce qu'on met en contact les acides peroxycarboxyliques en phase aqueuse, cette phase contenant de l'acide sulfurique, avec le sulfate de sodium, à l'occasion de quoi on fabrique au moins une partie du sulfate de sodium nécessaire pour la stabilisation par formation "in situ" à partir de l'acide sulfurique présent et d'un composé du sodium à caractère basique, de préférence la soude.

7. Procédé selon l'une quelconque des revendications 1 à 6, caractérisé en ce qu'on extrait la chaleur pour effectuer la cristallisation des sulfates de sodium hydratés de la lessive-mère par refroidissement par évaporation sous vide.

8. Procédé selon la revendication 7, caractérisé en ce qu'on règle le refroidissement par évaporation dans le haut de la zone de température, de préférence entre la température à laquelle on réalise la stabilisation et environ + 10°C.

9. Procédé selon la revendication 7 ou 8, caractérisé en ce qu'on réalise la cristallisation du sulfate de sodium hydraté en 2 ou plusieurs étapes, où l'on sépare après chaque étape le sulfate de sodium hydraté ayant cristallisé de la lessive-mère appauvrie en sulfate de sodium, et où l'on envoie cette lessivemère à l'étape suivante pour faire baisser la température de la lessive-mère et faire cristalliser le sulfate de sodium hydraté.

10. Procédé selon l'une quelconque des revendications 1 à 6, caractérisé en ce qu'on extrait la chaleur au moyen d'un échangeur de chaleur dans la partie basse de la zone de température, de préférence à partir d'environ + 10°C jusqu'à moins de 0°C, au voisinage du point cryohydrique, pour faire cristalliser le sulfate de sodium hydraté de la lessive-mère.

11. Procédé selon l'une quelconque des revendications 7 à 9 et 10, caractérisé en ce qu'on extrait d'abord la chaleur de la lessive-mère dans le haut de la zone de température par réfrigération, par évaporation sous vide, puis après cela dans le bas de la zone de température par échangeur de chaleur.

12. Procédé selon l'une quelconque des revendications 1 à 8 ou 10 à 11, caractérisé en ce qu'on réalise la cristallisation en une étape, où l'on prélève la suspension de cristaux de façon continue dans l'enceinte de cristallisation équipée d'un dispositif efficace de refroidissement, on sépare les cristaux, et on recycle la lessive-mère ainsi appauvrie en sulfate de sodium vers l'enceinte de cristallisation.

13. Procédé selon l'une quelconque des revendications 1 à 12, caractérisé en ce qu'on réalise la cristallisation du sulfate de sodium hydraté de la lessive-mère à une température à peu près constante, de préférence au-dessous de + 10°C et au-dessus du point cryohydrique, au moment où l'on transfère de façon continue vers l'enceinte de cristallisation équipée d'un dispositif de refroidissement par évaporation efficace et/ou d'un échangeur de chaleur, la lessive-mère saturée en sulfate de sodium, au-dessus de la température de cristallisation, au moment où l'on extrait de façon continue le sulfate de sodium décahydrate formé et/ou la suspension de cristaux de sulfate de sodium heptahydrate et au moment où l'on sépare les cristaux de la lessive résiduaire.

14. Procédé selon l'une quelconque des revendications 1 à 13, caractérisé en ce qu'on transforme les sulfates de sodium hydratés, séparés de la lessive résiduaire en les faisant fondre à une température égale ou supérieure à celle du point de transformation du sulfate de sodium décahydrate en Thenardite, de préférence entre 32,4°C et 45°C en une solution de sulfate de sodium saturée et contenant du sulfate de sodium anhydre en suspension, on sépare le sulfate de sodium en suspension de la solution par un procédé connu en soi, et on recycle au moins une partie de la solution et/ou du sulfate de sodium anhydre vers le process de stabilisation.

15. Procédé selon l'une quelconque des revendications 6 à 14, caractérisé en ce qu'on ajoute la solution saturée en sulfate de sodium obtenue par les sulfates de sodium hydratés, à la phase aqueuse contenant de l'acide sulfurique et les acides peroxycarboxyliques devant être stabilisés, on transforme l'acide sulfurique en sulfate de sodium par un procédé connu en soi et on ajoute le sulfate de sodium anhydre venant des sulfates de sodium hydratés totalement ou partiellement pour réaliser le conditionnement des acides peroxycarboxyliques stabilisés avant ou après la séparation de ces derniers de la lessive-mere.

16. Procédé selon l'une quelconque des revendications 1 à 15, caractérisé en ce qu'à côté du sulfate de sodium d'autres agents de stabilisation sont présents, particulièrement l'acide borique, les sulfates alcalins, alcalino-terreux et les sulfates de métaux terreux.